# EUROPÄISCHE PATENTSCHRIFT

(11) **EP 1 489 979 B1**
(45) Veröffentlichungstag und Bekanntmachung des Hinweises auf die Patenterteilung: **17.05.2006**
(21) Anmeldenummer: 03720150.6
(22) Anmeldetag: 08.03.2003
(51) Int. Cl.: A61B 17/66, A61B 17/80, A61C 8/00

(54) **DISTRAKTIONGSGERÄT FÜR DIE OSTEOGENESE**
DISTRACTION DEVICE USED FOR OSTEOGENESIS
APPAREIL DE DISTRACTION POUR OSTEOGENESE

(30) Priorität: 22.03.2002 DE 10212815
(43) Veröffentlichungstag der Anmeldung: 29.12.2004
(73) Patentinhaber: Fuchs, Ernst, 8800 Thalwil (CH); Cierny, Michael, 8802 Kilchberg (CH)
(72) Erfinder: Fuchs, Ernst, 8800 Thalwil (CH); Cierny, Michael, 8802 Kilchberg (CH)
(74) Vertreter: Pöhner, Wilfried Anton, Dr.
(86) Internationale Anmeldenummer: PCT/DE2003/000741
(87) Internationale Veröffentlichungsnummer: WO 2003/079912

(56) Entgegenhaltungen:
- WO-A-00/56235
- WO-A-97/20512
- DE-A- 19 537 023
- US-A- 5 810 812

## Beschreibung

Die Erfindung betrifft eine Vorrichtung zur vertikalen Kallusdistraktion mit einem eine Gewindespindel aufweisenden Trieb und zwei durch den Trieb relativ zueinander verschiebbaren Ankern von denen einer mit einem feststehenden Knochensegment und einer mit einem beweglichen Knochensegment verbindbar ist, wobei der Trieb als Lineartrieb ausgebildet ist, mittels dessen zwei Elemente ohne Drehung zueinander translatorisch zueinander verschiebbar sind, und der eine der genannten Anker am ersten Ende des einen Elementes und der andere der genannten Anker am ersten Ende des anderen Elementes festgelegt ist.

Ein Teilgebiet der Kiefer-, Mund- und Gesichtschirurgie umfaßt die Rekonstruktion von Knochenstrukturen. Derartige Maßnahmen sind bei einer Reihe von Erkrankungen gefordert, wie in schweren Fällen von Lippen-Kiefer-Gaumen-Spalten, aber auch Crouzon oder Apertsyndromen. Daneben ist auch die Behandlung von Knochendefekten nach Trauma oder Tumorchirurgie auf die Rekonstruktion von Knochen angewiesen.

Konventionelle Augmentationsverfahren beruhen auf der Transplantation von autologem Gewebe. Dabei gehören sowohl die Auflagerungsosteoplastik als auch die Interposition von Knochen- oder Knorpelgewebe zu den im Stand der Technik praktizierten Verfahren. Hierbei werden dem Patienten Knochenstückchen von Bereichen des Körpers entnommen, die außerhalb des Kopfes liegen, und an der defekten Stelle im Gesichts- oder Kiefernbereich eingesetzt. Bei der Rekonstruktion von Kiefer und Gebiß schließt sich in der Regel an die genannten Maßnahmen die Insertion von Implantaten an.

Die genannten Verfahren weisen jedoch eine Reihe von Nachteilen und Problemen auf, zu denen u.a. langsame Mineralisation und erhöhtes Infektionsrisiko der therapierten Körperstelle und eine vergleichsweise hohe Morbidität der Entnahmestelle gehören.

Ein neueres Verfahren zur Rekonstruktion von Knochenstrukturen ist unter dem Namen Distraktionosteogenese bekannt geworden. Anstelle einer Implantation von Gewebe wird bei diesem Verfahren Knochengewebe neu gewonnen. Die Grundlagen zu diesem Verfahren wurden durch den russischen Orthopäden Ilizarov im Jahr 1988 gelegt und führten zu einer Methode zur Verlängerung von Röhrenknochen. Diese beruht darauf, einen nach Kortikotomie gebildeten Kallus mit einer Geschwindigkeit von etwa einem Millimeter pro Tag auseinander zu ziehen. Hierdurch werden die Selbstheilungskräfte des Knochens aktiviert, so daß laufend neues Kallusgewebe produziert und dadurch der Röhrenknochen verlängert wird.

Die Distraktionsosteogenese hat sich zu einem bedeutenden Therapieverfahren in der Mund-, Kiefer- und Gesichtschirurgie entwickelt. Insbesondere wird sie eingesetzt, um zahnlose oder zahntragende Alveolarfortsatzsegmente und hochatrophe Unterkiefer im Front-, Seitenzahnbereich oder im Bereich des gesamten Unterkiefers vertikal aufzubauen. Bei der Durchführung des genannten Verfahrens kommen Distraktionsvorrichtungen zum Einsatz, welche die Aufgabe haben, ein den Rekonstruktionsbereich abdeckendes Knochensegment, das zuvor operativ vom Restknochen getrennt wurde, gegenüber dem Restknochen kontinuierlich zu verschieben. Dabei hat die permanente Verschiebung des Knochensegments zur Folge, daß der zwischen den Ufern des verschobenen und feststehenden Knochens sich bildende Kallus sich permanent vergrößert und somit neues Knochengewebe gewonnen wird. Dabei hat die Praxis gezeigt, daß die Bildung von Kallus um so intensiver ist, je kleiner die Distraktionsschritte sind, d.h. optimal ausfällt, wenn die Distraktion kontinuierlich erfolgt. Neben der Bildung von neuem Knochen ist bei dieser Methode auch die einhergehende Dehnung und Vermehrung des umliegenden Weichgewebes (Histogenese) von großem Vorteil. Sie trägt wesentlich zur Vitalität des verschobenen Knochensegmentes und damit zur Verminderung von Infektionsrisiken und zur schnellen Abheilung bei.

Eine Vorrichtung gattungsgemäßer Art ist DE 19 537 023 A 1 entnehmbar, in welcher flächige und am Knochen tangential anliegende Anker Verwendung finden, die über senkrecht hierzu verlaufende Schrauben im Knochenmaterial befestigt werden.

Ein wesentlicher Gesichtspunkt bei der Entwicklung von Distraktoren ist deren Miniaturisierung. Bei den im Stand der Technik bekannten Distraktoren führte diese Vorgabe jedoch in nachteiliger Weise zu einer mäßigen Stabilität der Vorrichtung und einer wenig exakten Führung der gegeneinander verschiebbaren Anker.

Vor diesem Hintergrund hat sich die Erfindung die Aufgabe gestellt, eine Vorrichtung zur Distraktionsosteogenese anzugeben, welche die genannten Nachteile vermeidet, und insbesondere eine exakte Führung der gegeneinander verschiebbaren Anker auch bei kleinen Abmessungen der Vorrichtung sicherstellt. Darüber hinaus erlaubt die vorgeschlagene Vorrichtung auch deren Verwendung als Zahnimplantatpfosten, welche im Kiefer des Patienten verbleiben.

Erfindungsgemäß wird diese Aufgabe dadurch gelöst, daß
- die Anker plattenförmig ausgeführte Bereich aufweisen,
- welche im wesentlichen senkrecht zur Verschiebungsrichtung orientiert sind.

Wie bei Distraktoren nach dem Stand der Technik weist vorliegender Distraktor einen Trieb und zwei durch den Trieb relativ zueinander verschiebbare Anker auf, wobei der Trieb als Lineartrieb ausgebildet. Diese Ausbildung stellt sicher, daß die beiden Hauptelemente des Triebs ohne Drehung zueinander rein translatorisch verschiebbar sind. Gemäß Vorschlag sind die genannten Anker jeweils an dem einen Ende der beiden Elemente festgelegt.

Bei Anwendung der Vorrichtung zur Distraktionsosteogenese wird der eine Anker mit dem verschiebbaren Knochensegment verbunden, der andere mit dem feststehenden Knochen. Die dem Trieb eigene exakte Führung überträgt sich damit auch auf die beiden Anker und somit auf die Relativbewegung zwischen den beiden Knochen. Die Distraktion erfolgt daher in einer genau vorgegebenen Richtung. Die vorteilhafte Folge hiervon ist, daß die Form des gebildeten Kallus in jedem Zeitpunkt der Anwendung der geplanten Form entspricht. Der aus dem Kallus erwachsende neue Knochenbereich weist daher nach Abschluß der Behandlung genau die geplante Form und Größe auf.

Die rein translatorische Verschiebung der Anker ohne gleichzeitige Drehung der mit diesen verbundenen Elemente des Lineartriebes bringt noch einen weiteren Vorteil mit sich. Bei einer Distraktionosteogenese wird häufig die komplette Distraktionsvorrichtung implantiert, bis auf den Verstellkopf der Vorrichtung, der bei vorliegender Vorrichtung am zweiten Ende der beiden genannten Elemente gelegen ist. Bei Vorrichtungen nach dem Stand der Technik bedeutet dies, daß die Gewindewelle zur Verschiebung der Anker ebenfalls implantiert ist und bei Verstellung der Anker relativ zum umgebenden Gewebe verdreht werden muß. Bei der erfindungsgemäßen Vorrichtung hingegen ist zur Verschiebung der Anker lediglich eine Verschiebung eines Elementes mit glatter Oberfläche relativ zum umgebenden Gewebe notwendig.

Die durch eine exakte Führung des Lineartriebes erzielte Wirkung wird bei vorliegenden Vorrichtung noch durch eine geeignete Ausbildung der Anker unterstützt. Es ist vorgesehen, daß die Anker plattenförmig ausgeführte Bereiche aufweisen, welche im wesentlichen senkrecht zur Verschiebungsrichtung orientiert sind. Diese Ausbildung gewährleistet in vorteilhafter Weise eine exakte Abstützung des einen Ankers am feststehenden Knochen und eine exakte Unterstützung des zu verschiebenden Knochensegmentes durch den anderen Anker vor allem gegen Kippmomente. Die Verschiebung des beweglichen Knochensegmentes erfolgt bei letzt genannter Ausführungsform des Distraktors senkrecht zu den plattenförmigen Bereichen der Anker und ist somit exakt vorbestimmt.

Ein weiterer Vorteil letztgenannter Ausführungsform ist darin zu sehen, daß für die linke und rechte Kiefemhälfte sowohl bei Anwendung im Ober- als auch Unterkiefer ein und dieselbe Ausführung einsetzbar ist. Bei Distraktoren nach dem Stand der Technik hingegen ist für die genannten Einsatzbereiche in der Regel ein "rechter" und ein "linker" Distraktor erforderlich.

Bei der Ausgestaltung der plattenförmig ausgeführten Bereiche der Anker sieht die Erfindung mehrere vorteilhafte Lösungen vor.

Eine erste Lösung weist Anker auf, bei denen die plattenförmig ausgeführten Bereiche exzentrisch zum Lineartrieb angeordnet sind. Diese Ausgestaltung führt dazu, daß der Lineartrieb im Randbereich des Ankers zu liegen kommt. Sie ermöglicht eine vorteilhafte Anwendung des Distraktors, bei welcher der plattenförmig ausgebildete Bereich wenigstens eines der beiden Anker in einen von außen in das betreffende Knochensegment führenden Schlitz eingeführt ist, während der Lineartrieb außerhalb des Knochens verbleibt.

Der Vorteil dieser Ausführung ist darin zu sehen, daß der Distraktor mit vergleichsweise wenig Aufwand von außen angebracht und entfernt werden kann. Dieser Art der Incorporation führt darüber hinaus dazu, daß der plattenförmig ausgebildete Bereich des betreffenden Ankers einen sehr stabilen Sitz im Knochen erhält. Die Distraktionsrichtung bei der genannten Anwendung liegt senkrecht zur Orientierung des Schlitzes und ist - da die Orientierung des Schlitzes sehr genau vorgebbar ist - in vorteilhafter Weise ebenfalls sehr exakt vorbestimmbar.

Bei einer vorteilhaften Weiterbildung der genannten Ausführungsform ist vorgesehen, daß sich die Dicke des plattenförmig ausgeführten Bereichs wenigstens eines Ankers wenigstens an den vom Lineartrieb wegweisenden Rändern keilförmig verjüngt. Beim Einsetzen des betreffenden Ankers in den Knochenschlitz werden die keilförmigen Ränder dann in die Knochensubstanz eingedrückt. Hierdurch erhält der Sitz Ankers im Knochengewebe eine noch höhere Stabilität als bei vorbenannter Ausführung.

Der gleichen Zielsetzung, dem Distraktor nach dessen Inkorporation in das Knochengewebe einen stabilen Sitz zu verleihen, dient auch eine weitere Ausgestaltung der Erfindung. Hierbei ist vorgesehen, auch die Außenfläche des Lineartriebes in der zu den exzentrisch angeordneten plattenförmigen Bereichen weisenden Richtung keilförmig auszubilden. Die keilförmige Gestalt der genannten Außenfläche wird bei Inkorporation des Distraktor dann gegen die Oberfläche des betreffenden Knochensegmentes gepreßt und verbessert hierdurch ebenfalls den Sitz des Distraktors.

Eine entscheidende Voraussetzung für die Anwendbarkeit von Distraktoren im Kiefernbereich sind kleine Abmessungen der Distraktorelemente. Etliche Maßnahmen bei der Entwicklung von Distraktoren sind daher auf deren Miniaturisierung ausgerichtet. Eine weitere Lösung zur Ausgestaltung der Anker verfolgt eben diese Zielsetzung. Sie ist dadurch gekennzeichnet, daß der plattenförmig ausgeführte Bereich des einen Ankers innerhalb einer Aussparung des plattenförmig ausgeführten Bereichs des anderen Ankers Platz findet. Im vollkommen eingefahrenen Zustand des Distraktors liegen beide Anker daher nicht aufeinander, sondern in einander und verursachen somit nur eine Aufbauhöhe von der einfachen Dicke des plattenförmigen Bereichs. Distraktoren diese Bauart lassen sich somit innerhalb eines Osteomie-Schnittes geringer Höhe einsetzen.

Bei der Ausbildung des Lineartriebs wird gemäß vorliegender Erfindung eine Lösung bevorzugt, bei welcher die beiden verschiebbaren Elemente teleskopisch in einander laufen. Die Erfindung sieht hierbei zwei Varianten vor, eine erste bei der beide Elemente einen unrunden, vorzugsweise quadratischen, Querschnitt aufweisen, und eine zweite, bei der beide Elemente einen runden Querschnitt besitzen. Bei letzterer Variante ist eines der Elemente mit einer Längsnut ausgestattet, in die ein mit dem anderen Element starr verbundener Zapfen eingreift. Dabei ist es gleichwertig, an welchem Element die Nut vorgesehen und an welchem Element der Zapfen festgelegt ist. Der Vorteil der vorgeschlagenen Ausführung liegt darin, daß sie einerseits eine exakte Führung zur Verfügung stellt, anderseits mit Blick auf eine Miniaturisierung auch kleine Abmessungen des Triebes zuläßt.

Die Verstellung des Lineartriebes wird vom zweiten Ende der beiden Elemente aus durchgeführt. Bei der Ausgestaltung des Verstellkopfes sieht die Erfindung zwei Varianten vor.

Bei der einen Variante ist das äußere der beiden Elemente im Bereich von dessen zweiten Ende mit einem Innengewinde ausgestattet, in welches eine Mutter mit Außengewinde eingreift. Die Mutter ihrerseits weist ein konzentrisch angeordnetes Sackloch auf, welches das zweite Ende des inneren Elementes mit einem geringen Spiel übergreift. Im Falle eines Rechtsgewindes führet eine Drehung der Mutter im Uhrzeigersinn dazu, daß die Mutter kontinuierlich im Innengewinde verschwindet. Da sie sich jedoch auf dem in das Sackloch eingreifenden zweiten Ende des inneren Elementes abstützt, wird bei diesem Vorgang das äußere Element kontinuierlich in Richtung zu dessen zweiten Ende hin verschoben. Die an den beiden ersten Enden der Elemente jeweils befestigten Anker werden dabei voneinander weg bewegt und somit der Abstand des frei beweglichen Knochensegmentes gegenüber dem feststehenden Knochen kontinuierlich vergrößert.

Bei der anderen Variante sind die Rollen von innen- und außen liegendem Element vertauscht. Dementsprechend weist das innere der beiden Elemente im Bereich seines zweiten Endes ein Außengewinde auf, in welches eine Mutter mit Innengewinde eingreift. Die Mutter ist ihrerseits drehbar am äußeren Element festgelegt. Im Falle eines Rechtsgewindes führt eine Drehung der Mutter im Gegenuhrzeigersinn dazu, daß die Mutter und damit das an ihr festgelegte äußere Element kontinuierlich in Richtung zu dessen zweiten Ende hin verschoben wird. Die damit verbunden Relativbewegungen der an den Elementen festgelegten Anker entsprechen denen der vorbenannten Variante.

Zur Drehung der Mutter bei vorliegender Distraktionsvorrichtung sind an der von den genannten Elementen wegweisenden Stirnseite der Mutter entsprechende Mittel vorgesehen. Sie umfassen beispielsweise einen Sechskant für einen Mutternschlüssel und/oder einen Schlitz für einen Schraubendreher.

Die Befestigung der Anker am ersten Ende der genannten Elemente des Lineartriebes läßt mehrere konstruktive Möglichkeiten zu. Von Vorteil für den Einsatz der erfindungsgemäßen Vorrichtung zur Osteogenese ist eine Festlegung der Anker jeweils mittels eines Gewindes am ersten Ende des jeweiligen Elementes. Das Gewinde gewährleistet einerseits eine sichere und biegesteife Verbindung, läßt jedoch die Möglichkeit zu, die Anker und die genannten Elemente des Lineartriebs von einander zur trennen. Letztere Option ist dann von Interesse, wenn die Komponenten der Distraktionsvorrichtung nach Abschluß der Behandlung wieder entfernt werden sollen. In diesem Fall wird die Schraubverbindung zwischen Ankern und Linearelementen gelöst um anschließend die Komponenten der Vorrichtung aus dem Behandlungsbereich zu entfernen.

Bei Ausbildung der Ankerbefestigung an den Elementen des Lineartriebes als lösbare Verbindung ist es zweckmäßig, wenn der Drehsinn der vorhandenen Gewinde jeweils geeignet aufeinander abgestimmt ist. Dementsprechend wird bei der Variante der Vorrichtung, die eine Verstellmutter mit Außengewinde aufweist, vorgeschlagen, das Gewinde der genannten Mutter und der genannten Anker jeweils gleichsinnig, vorzugsweise jeweils als Rechtsgewinde, auszubilden. Bei der Variante der Vorrichtung, die eine Verstellmutter mit Innengewinde aufweist, ist es dagegen zweckmäßig, wenn das Gewinde der genannten Mutter und der genannten Anker jeweils gegensinnig ausgeführt sind. Bevorzugt wird hierbei eine Lösung, bei der das Gewinde der Mutter als Rechtsgewinde und das Gewinde der genannten Anker jeweils als Linksgewinde ausgebildet ist.

Diese Ausführungen stellen sicher, daß bei einer Drehung der Mutter in der Richtung, welche zu einer Vergrößerung des Abstandes zwischen beiden Ankern führt, jeweils die von der Mutter auf die Elemente übertragenen Momente ein Einschrauben der genannten Elemente in die Anker begünstigen. Ein selbsttätiges Lösen der Verbindung zwischen Ankern und Elementen des Lineartriebes ist bei einer Vergrößerung der Distraktionszone daher ausgeschlossen. Will man dagegen nach Abschluß der Behandlung die Elemente des Lineartriebes entfernen, genügt ein Drehen in Linksrichtung, um die Verbindung zwischen den genannten Elementen und den Ankern jeweils zu lösen.

Der Anwendung der Distraktionsvorrichtung zur Implantation im menschlichen Körper entsprechend ist das Material der Vorrichtung aus Edelmetall ausgebildet. Insbesondere ist gemäß einem Merkmal der Erfindung vorgesehen, die Komponenten aus Gold oder Platin herzustellen.

Die Erfindung sieht sowohl einen Einsatz der vorgeschlagenen Vorrichtung vor, bei der diese nach Abschluß der Distraktion wieder entfernt wird, als auch eine Anwendung, bei welcher die Vorrichtung oder Komponenten davon im Körper des Patienten verbleiben. Letzterer Fall ist von Interesse, wenn die Osteogenese mit einer nachfolgenden Rekonstruktion des Gebisses verbunden ist. In diesem Fall wird vorgeschlagen, die Komponenten der Distraktionsvorrichtung als Zahnimplantatpfosten zu nutzen und Zahnprothesen darauf aufzubauen. Der Vorteil dieser Lösung ist offenkundig, sie erspart weitere der Herstellung des Gebisses dienende Eingriffe in den Kiefer nach Abschluß der Distraktionsbehandlung.

Bei Distraktionsvorrichtungen, welche nach Abschluß der Distraktion im Körper des Patienten verbleiben, werden seitens der Erfindung Ausführungsformen empfohlen, bei denen die Anker zentrisch zum Lineartrieb ausgebildet sind. Bevorzugt werden hierbei Anker mit tellerartiger Geometrie, wobei innerhalb des Tellers in der Regel Aussparungen vorgesehen sind, welche bezüglich der Achse des Lineartriebes eine zwei-, drei- oder mehrzählige Symmetrie besitzen. Bei minimalem Knochenangebot kommen bevorzugt Anker mit sternförmiger Geometrie zum Einsatz, die ebenfalls eine zwei-, drei- oder mehrzählige Symmetrie aufweisen.

Bei Distraktionsvorrichtungen, die nach erfolgter Distraktion wieder entfernt werden, ist es hingegen von Vorteil, wenn - wie oben bereits erwähnt - die Anker exzentrisch zum Lineartrieb angeordnet und als plattenförmige Elemente ausgebildet sind. Der plattenförmige Bereich der Anker ist hierbei vorzugsweise jeweils in Form eines U's ausgebildet.

Unabhängig von der zentrischen oder exzentrischen Anordnung sieht die Erfindung unterschiedliche Arten zur Festlegung der Anker vor. Bei einer ersten Variante sind die plattenförmig ausgebildeten Bereiche beider Anker in der Trennfuge zwischen beweglichem und fest stehendem Knochensegment angeordnet sind. Eine weitere Anwendung ist dadurch gekennzeichnet, daß der plattenförmig ausgebildete Bereich wenigstens eines der beiden Anker in das jeweilige Knochensegment inkorporiert ist. Ein besonders stabiler Sitz der Vorrichtung zur Distraktion läßt sich hierbei insbesondere dann erreichen, wenn beide Anker innerhalb des Knochengewebes inkorporiert sind.

Weitere Einzelheiten, Merkmale und Vorteile der Erfindung lassen sich dem nachfolgenden Teil der Beschreibung entnehmen. In diesem Teil wird ein Ausführungsbeispiel der erfindungsgemäßen Vorrichtung zur Distraktionsosteogenese anhand einer Zeichnung erläutert. Gleiche Bezugszeichen in den einzelnen Figuren bezeichnen dabei jeweils gleiche Elemente. Im einzelnen zeigen:
- Figur 1a:: einen Längsschnitt durch die Vorrichtung
- Figur 1b:: einen Schnitt entsprechend der Schnittebene AB nach Figur 1 a.
- Figur 2:: einen Längsschnitt durch eine Variante der Vorrichtung

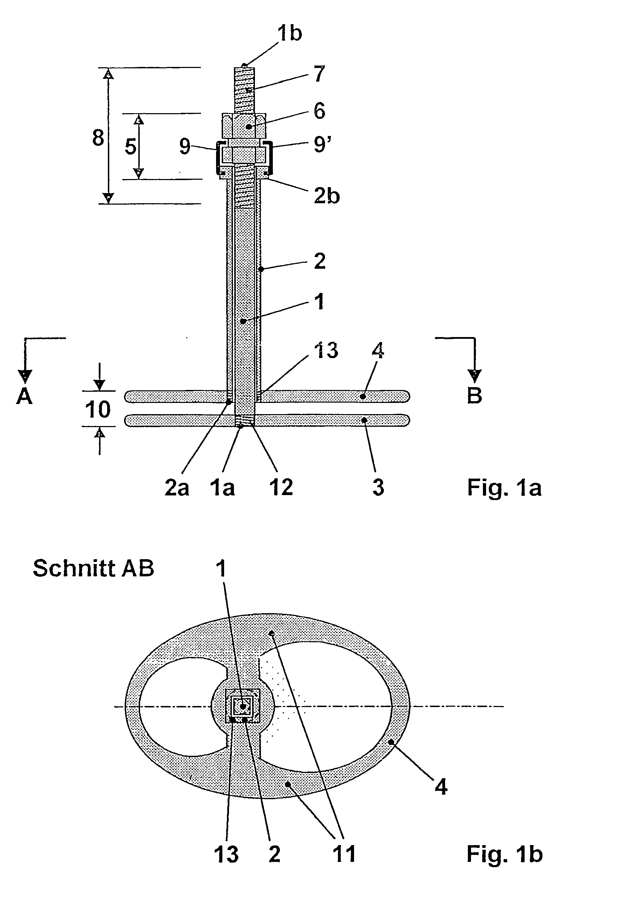

Der Figur 1 läßt sich der prinzipielle Aufbau des Distraktors entnehmen. Die wesentlichen Komponenten umfassen einen Lineartrieb mit den Elementen 1 und 2 und die an deren jeweiligen ersten Ende 1 a, 2a befestigten Anker 3 und 4. Bei vorliegender Ausführungsform laufen die Elemente 1, 2 teleskopisch ineinander, wobei Element 1 das innere, Element 2 das äußere Element bildet. Beide Elemente weisen einen unrunden Querschnitte auf, Element 1 einen quadratischen und Element 2 den eines quadratischen Vierkantrohres. Die gewählte Ausbildung des Lineartriebes stellt sicher, daß die beiden Elemente 1, 2 des Triebs ohne Drehung zueinander rein translatorisch verschiebbar sind.

Die Verstellung des Lineartriebes wird mittels des Verstellkopfes 5 vom zweiten Ende 1b, 2b der beiden Elemente 1, 2 aus durchgeführt. Der Verstellkopf 5 umfaßt eine Mutter 6, die mit einem Innengewinde ausgestattet ist und auf dem inneren Element 2 aufgeschraubt ist. Das Element 2 weist daher im Bereich 8 seines zweiten Endes 2b ein entsprechendes Außengewinde 7 auf. Die Mutter 6 ist ihrerseits drehbar am zweiten Ende 2b des äußeren Elementes 2 festgelegt. Die Festlegung der genannten Art wird in vorliegender Darstellung durch die beiden Klammern 9, 9' angedeutet. Bei dem zugrunde gelegten Rechtsgewinde 7 führt eine Drehung der Mutter 6 im Gegenuhrzeigersinn dazu, daß die Mutter 6 und damit das an ihr festgelegte äußere Element 2 kontinuierlich in Richtung zum Ende 1 b des ersten Elementes 1 hin verschoben wird. Die an den beiden ersten Enden 1 a, 2a der Elemente 1, 2 jeweils befestigten Anker 3, 4 werden hierbei voneinander weg bewegt und somit der Abstand 10 zwischen ihnen vergrößert.

Bei Anwendung der Vorrichtung zur Distraktionsosteogenese wird der eine Anker 4 mit dem (nicht dargestellten) verschiebbaren Knochensegment verbunden, der andere 3 mit dem (nicht dargestellten) feststehenden Knochen. Die exakte Führung des Lineartriebes hat hierbei eine ebenso exakte Bewegung des verschiebbaren Knochensegmentes zur Folge. Bei Durchführung der Distraktion führt eine Vergrößerung des Abstandes 10 zwischen beiden Ankern 3, 4 dann dazu, daß der zwischen den Ufern des verschobenen und feststehenden Knochens entstehende Kallus sich permanent vergrößert und somit neues Knochengewebe gewonnen wird.

Wie der Figur 1 zu entnehmen ist, weisen die Anker 3, 4 plattenförmig ausgeführte Bereiche 11 auf, die eine exakte Abstützung des einen Ankers 3 am feststehenden Knochen und eine exakte Unterstützung des zu verschiebenden Knochensegmentes durch den anderen Anker 4 gewährleisten.

Bei vorliegender Ausführung sind die Anker 3, 4 jeweils mittels eines Gewindes 12, 13 am ersten Ende 1 a, 2a des jeweiligen Elementes 1, 2 befestigt. Diese Art der Befestigung läßt die Möglichkeit zu, die Anker 3, 4 und die Elemente 1, 2 des Lineartriebs von einander zur trennen und ist dann zu empfehlen, wenn die Komponenten der Distrktraktionsvorrichtung nach Abschluß der Behandlung wieder entfernt werden sollen.

Bei Vorgabe eines Rechtsgewindes 7 für die Mutter 6 ist für die Ankerbefestigung jeweils ein Linksgewinde 12, 13 vorzusehen. Diese Ausführung stellt sicher, daß bei einer Drehung der Mutter 6 in Gegenuhrzeigersinn zur Vergrößerung des Abstandes 10 zwischen beiden Ankern 3, 4 die von der Mutter auf die Elemente 1, 2 übertragenen Momente nicht zu einem Lösen der Verbindung zwischen Ankern 3, 4 und Elementen 1, 2 des Lineartriebes führen.

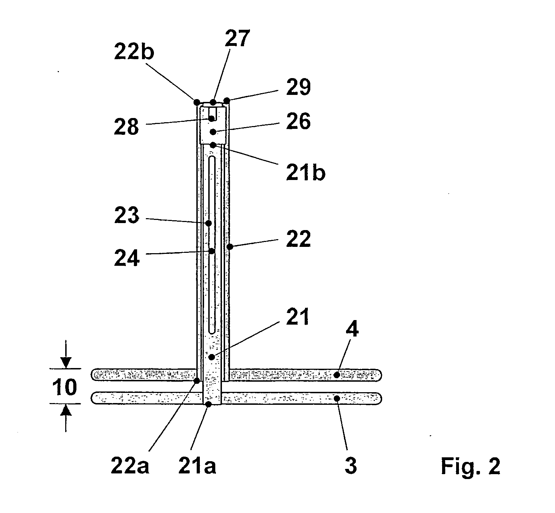

Figur 2 gibt eine Variante der Vorrichtung wieder, bei welcher der Lineartrieb eine Hülse 22 mit rundem Innenquerschnitt und einen teleskopisch darin laufenden Stift 21 mit rundem Außenquerschnitt aufweist. Eine Längsnut 23 im Stift 21, in welche ein mit der Hülse 22 starr verbundener Zapfen 24 eingreift, sorgt dafür, daß sich beide Elemente 21, 22 beim Verstellen des Lineartriebes nicht gegeneinander verdrehen. Die Verstellung des Lineartriebes wird durch eine Mutter 25 bewirkt, welche auf dem zweiten Ende 21 b des Stiftes 21 aufsitzt und mit einem Innengewinde der Hülse 22 kämmt. Das Außengewinde der Mutter 25 und das Innengewinde der Hülse 22 sind in vorliegender Zeichnung der Übersichtlichkeit halber nicht eingezeichnet. Die Mutter 25 wird durch einen (nicht eingezeichneten) Schraubendreher betätigt, welcher durch eine am zweiten Ende 22b der Hülse 22 stirnseitig angebrachte Bohrung 26 hindurch in einen Schlitz 27 in der Mutter 25 eingreift. Der Durchmesser der Bohrung 26 ist dabei so bemessen, daß an der Hülse ein die Mutter 25 übergreifender Kragen 28 erhalten bleibt.

Bei einem Rechtsgewinde an Mutter 25 und Hülse 22 führt (bei Blick auf den Mutternschlitz) eine Verdrehung der Mutter 25 im Uhrzeigersinn dazu, daß die Mutter 25 die Hülse 22 und damit den an ihrem ersten Ende 22a befestigten Anker 4 anhebt und somit den Abstand 10 zwischen beiden Ankern 3 und 4 vergrößert.

## Patentansprüche

1. Vorrichtung zur vertikalen Kallusdistraktion mit einem eine Gewindespindel aufweisenden Trieb und zwei durch den Trieb relativ zueinander verschiebbaren Ankern (3,4) von denen einer mit einem feststehenden Knochensegment und einer mit einem beweglichen Knochensegment verbindbar ist,
wobei
der Trieb als Lineartrieb ausgebildet ist,
mittels dessen zwei Elemente (1,2) ohne Drehung zueinander translatorisch zueinander verschiebbar sind,
und der eine der genannten Anker (3) am ersten Ende (1a) des einen Elementes (1)
und der andere der genannten Anker (4) am ersten Ende (2a) des anderen Elementes (2) festgelegt ist und
die Anker (3,4) plattenförmig ausgeführte Bereiche (11) aufweisen, **dadurch gekennzeichnet, dass** die Normalen-Vektoren der Anker (3, 4)
im wesentlichen parallel zur Verschiebungsrichtung orientiert sind.

2. Vorrichtung nach Anspruch 1, **dadurch gekennzeichnet, daß**
- die plattenförmig ausgeführten Bereiche (11) exzentrisch zum Lineartrieb angeordnet sind.

3. Vorrichtung nach Anspruch 2, **dadurch gekennzeichnet, daß**
- sich die Dicke des plattenförmig ausgeführten Bereichs (11) wenigstens eines Ankers (3 bzw. 4) wenigstens an den vom Lineartrieb wegweisenden Rändern keilförmig verjüngt.

4. Vorrichtung nach Anspruch 2 oder 3, **dadurch gekennzeichnet, daß**
- die Außenfläche des Lineartriebes in der Richtung der genannten Exzentrizität keilförmig ausgebildet ist.

5. Vorrichtung nach einem der Ansprüche 1-4, **dadurch gekennzeichnet, daß**
- der plattenförmig ausgeführte Bereich (11) des einen Ankers (4) innerhalb einer Aussparung des plattenförmig ausgeführten Bereichs des anderen Ankers (3) Platz findet.

6. Vorrichtung nach einem der vorhergehenden Ansprüche, **dadurch gekennzeichnet, daß**
- die beiden genannten Elemente (1, 2) teleskopisch in einander laufen, und
- entweder beide Elemente (1, 2) einen unrunden, vorzugsweise einen quadratischen, Querschnitt aufweisen,
- oder beide Elemente (1, 2) einen runden Querschnitt besitzen
- und das eine der Elemente eine Längsnut aufweist,
- in die ein mit dem anderen Element starr verbundener Zapfen eingreift.

7. Vorrichtung nach einem der vorhergehenden Ansprüche, **dadurch gekennzeichnet, daß**
- das äußere der beiden Elemente (2) im Bereich seines zweiten Endes (2b) ein Innengewinde aufweist,
- in welches eine Mutter mit Außengewinde eingreift,
- die ein konzentrisch angeordnetes Sackloch aufweist,
- welches das zweite Ende des inneren Elementes mit einem geringen Spiel übergreift,
- und die genannte translatorische Bewegung durch Drehen an der Mutter erzeugbar ist.

8. Vorrichtung nach einem der vorhergehenden Ansprüche, **dadurch gekennzeichnet, daß**
- das innere der beiden Elemente (1) im Bereich seines zweiten Endes (1 b) ein Außengewinde (7) aufweist,
- in welches eine Mutter (6) mit Innengewinde eingreift,
- die drehbar am äußeren Element (2) festgelegt ist,
- und die genannte translatorische Bewegung durch Drehen an der Mutter (6) erzeugbar ist.

9. Vorrichtung nach einem der vorhergehenden Ansprüche, **dadurch gekennzeichnet, daß**
- Mittel zum Drehen der Mutter vorgesehen sind,
- beispielsweise ein Sechskant für einen Mutternschlüssel
- und/oder ein Schlitz für einen Schraubendreher,
- wobei die Mittel an der von den genannten Elementen wegweisenden Stirnseite der Mutter angebracht sind.

10. Vorrichtung nach einem der vorhergehenden Ansprüche, **dadurch gekennzeichnet, daß**
- die genannten Anker (3, 4) jeweils mittels eines Gewindes (12, 13) am ersten Ende (1a bzw. 2a) des jeweiligen Elementes (1 bzw. 2) festgelegt sind.

11. Vorrichtung nach einem der vorhergehenden Ansprüche, **dadurch gekennzeichnet, daß**
- das Gewinde der genannten Mutter (6) und der genannten Anker (3, 4) jeweils gleichsinnig, vorzugsweise jeweils als Rechtsgewinde, ausgeführt ist.

12. Vorrichtung nach einem der vorhergehenden Ansprüche, **dadurch gekennzeichnet, daß**
- das Gewinde der genannten Mutter (6) und der genannten Anker (3, 4) jeweils gegensinnig ausgeführt ist, vorzugsweise
- das Gewinde der genannten Mutter als Rechtsgewinde
- und das Gewinde (12, 13) der genannten Anker jeweils als Linksgewinde.

13. Vorrichtung nach einem der vorhergehenden Ansprüche, **dadurch gekennzeichnet, daß**
- die Komponenten der Vorrichtung
- aus Edelmetall,
- insbesondere aus Gold oder Platin
hergestellt sind.

## Claims

1. Device for vertical callous distraction comprising a drive having a lead-screw and two anchors (3, 4) which are displaceable relative to one another by means of the drive, of which one can be connected to a fixed bone segment and one to a movable bone segment
- the drive being designed as a linear drive,
- by means of which two elements (1, 2) can be translationally displaced with respect to one another, without rotation,
- and one of the aforementioned anchors (3) being fixed on the first end (1a) of one element (1)
- and the other of the aforementioned anchors (4) being fixed on the first end (2a) of the other element (2), and the anchors (3, 4) having plate-shaped regions (1), **characterised in that**
- the normal vectors of the anchors (3, 4) are oriented essentially parallel to the displacement direction.

2. Device according to claim 1, **characterised in that**
- in which the plate-shaped regions (11) are arranged eccentrically to the linear drive.

3. Device according to claim 2, **characterised in that**
- the thickness of the plate-shaped region (11) of at least one anchor (3 and/or 4) tapers in a wedge-shaped way at the edges facing away from the linear drive.

4. Device according to claim 2 or 3,
**characterised in that**
- the outer surface of the linear drive is designed as a wedge shape in the direction facing the said eccentricity.

5. Device according to one of claims 1-4, **characterised in that**
- the plate-shaped region (11) of one anchor (4) finds space within a recess of the plate-shaped region of the other anchor (3).

6. Device according to one of the preceding claims, **characterised in that**
- the said two elements (1, 2) run telescopically one inside the other, and
- either both elements (1, 2) have a non-circular, preferably square cross-section,
- or both elements (1, 2) have a circular cross-section,
- and one of the elements is equipped with a longitudinal groove,
- in which a peg, which is rigidly connected to the other element, engages.

7. Device according to one of the preceding claims, **characterised in that**
- the outer of the two elements (2) is equipped with an internal thread in the region of its second end (2b),
- in which a nut with an external thread engages,
- which nut has a concentrically arranged blind hole,
- which covers over the second end of the inner element with low play. -
- and the said translational displacement can be generated by rotation on the nut.

8. Device according to one of the preceding claims, **characterised in that**
- the inner of the two elements (1) has, in the region of its second end (1b), an external thread (7),
- in which a nut (6) with an internal thread engages, said nut is in turn fixed so as to be rotatable on the outer element (2).
- and the said translational displacement can be generated by rotation on the nut (6).

9. Device according to one of the preceding claims, **characterised in that**
- means for rotating the nut are provided,
- for example, a hexagon for a nut key
- and/or a slit for a screwdriver,
- the means being provided on that face side of the nut facing away from said elements.

10. Device for distraction osteogenesis according to one of the preceding claims, **characterised in that**
- the aforementioned anchors (3, 4) are in each case fixed by means of a screw thread (12, 13) at the first end (1a or 2a respectively) of the respective element (1 or 2 respectively).

11. Device for distraction osteogenesis according to one of the preceding claims, **characterised in that**
- the thread of the aforementioned nuts (6) and of the aforesaid anchors (3, 4) are in each case designed so as to rotate in the same direction, preferably as a right-hand thread.

12. Device according to one of the preceding claims, **characterised in that**
- the thread of the aforesaid nuts (6) and the aforesaid anchors (3, 4) are in each case designed so as to rotate in the same direction, preferably
- the thread of the said nuts as a right-hand thread and the thread (12, 13) of the said anchors in each case as a left-hand thread.

13. Device according to one of the preceding claims, **characterised in that**
- the components of the device are made of noble metal,
- in particular gold or platinum.

## Revendications

1. Dispositif pour la distraction verticale d'un cal avec une tige de poussée présentant une broche filetée et deux ancres (3, 4) pouvant être poussées relativement l'une par rapport à l'autre par la tige de poussée, dont l'une peut être reliée à un segment d'os fixe et une avec un segment d'os mobile,
sachant que
la tige de poussée est réalisée sous forme de tige de poussée linéaire,
à l'aide duquel deux éléments (1, 2) peuvent être déplacés sans rotation en translation l'un par rapport à l'autre,
et une des ancres citées (3) est fixée sur la première extrémité (1a) d'un des éléments (1)
et l'autre ancre citée () est fixée sur la première extrémité (2a) de l'autre élément (2) et
**caractérisé par le fait que**
les ancres (3, 4) présentent des zones (11) exécutées en forme de plaque, les vecteurs normaux des ancres (3, 4) étant orientés parallèlement à la direction de déplacement.

2. Dispositif selon la revendication 1, **caractérisé par le fait que**
1. les zones (11) exécutées en forme de plaque de façon excentrée par rapport à la tige de poussée linéaire.

3. Dispositif selon la revendication 2, **caractérisé par le fait que**
• l'épaisseur de la zone (11) exécutée en forme de plaque d'au moins une ancre (3 ou 4) s'amincit en forme de coin sur les bords n'étant pas tournés vers la tige de poussée linéaire.

4. Dispositif selon la revendication 2 ou 3, **caractérisé par le fait que**
• la surface extérieure de la tige de poussée linéaire est exécutée en forme de coin en direction de l'excentricité citée.

5. Dispositif selon l'une des revendications 1 - 4, **caractérisé par le fait que**
la zone exécutée en forme de plaque (11) d'une des ancres (4) trouve place à l'intérieur d'une réservation de la zone exécutée en forme de plaque de l'autre ancre (3).

6. Dispositif selon une des revendications précédentes **caractérisé par le fait que**
• les deux éléments cités (1, 2) courent de façon télescopique l'un dans l'autre, et
• soit les deux éléments (1, 2) présentent une section non ronde, de préférence carrée,
• ou les deux éléments (1, 2) une section ronde
• l'un des éléments présentant une rainure longitudinale,
• dans laquelle a prise un pivot relié de façon rigide avec l'autre élément.

7. Dispositif selon une des revendications citées précédemment, **caractérisé par le fait que**
• l'extérieur des deux éléments (2) présente un filetage intérieur dans la zone de sa deuxième extrémité (2b),
• dans lequel vient se prendre un écrou avec filetage extérieur,
• présentant un trou borgne disposé de façon concentrique,
• qui a prise sur la seconde extrémité de l'élément intérieur avec un faible jeu,
• le mouvement de translation cité pouvant être créé en tournant l'écrou.

8. Dispositif selon une des revendications précédentes **caractérisé par le fait que**
• l'intérieur des deux éléments (1) présente un filetage extérieur (7) dans la zone de sa seconde extrémité (1b),
• dans laquelle vient se prendre un écrou (6) avec un filetage intérieur,
• qui est fixé sur l'élément extérieur (2) de façon pivotante,
• et le mouvement de translation cité peut être créé en tournant sur l'écrou.

9. Dispositif selon une des revendications précédentes, **caractérisé par le fait que**
• des moyens sont prévus pour tourner l'écrou,
• par exemple six pans pour une clé d'écrou,
• et / ou une fente pour un tournevis,
• sachant que les moyens sont disposés sur le côté frontal de l'écrou n'étant pas tourné vers les éléments cités.

10. Dispositif selon une des revendications précédentes, **caractérisé par le fait que**
• les ancres citées (3, 4) sont chacune fixées au moyen d'un filetage (12, 13) sur la première extrémité (1a ou 2a) de chacun des éléments (1 ou 2).

11. Dispositif selon une des revendications précédentes **caractérisé par le fait que**
• le filetage de l'écrou cité (6) et de l'ancre citée (3, 4) est exécuté dans chaque cas en sens inverse, de préférence dans chaque cas avec un filet à droite.

12. Dispositif selon une des revendications précédentes, **caractérisé par le fait que**
• les filetages de l'écrou cité (6) et de l'ancre citée (3, 4) sont chacun exécutés en sens inverse, de préférence
o le filetage de l'écrou cité sous forme de filet à droite et le filetage (12, 13) des ancres citées sous forme d'un filet à gauche.

13. Dispositif selon une des revendications précédentes, **caractérisé par le fait que**
• les composants du dispositif sont composés
• en métal noble
• notamment en or ou en platine.
